Europäisches Patentamt

European Patent Office

Office européen des brevets

(19)

(11) Publication number: **0 151 513**

**A1**

(12)

# EUROPEAN PATENT APPLICATION

(21) Application number: **85300197.2**

(22) Date of filing: **11.01.85**

(51) Int. Cl.⁴: **C 07 C 103/28**
**A 61 K 31/165**

(30) Priority: **12.01.84 US 570143**

(43) Date of publication of application:
**14.08.85 Bulletin 85/33**

(84) Designated Contracting States:
**BE CH DE FR GB IT LI LU NL SE**

(71) Applicant: **ELI LILLY AND COMPANY**
**Lilly Corporate Center**
**Indianapolis Indiana 46285(US)**

(72) Inventor: **Schmiegel, Klaus Kurt**
**4507 Staughton Drive**
**Indianapolis Indiana 46226(US)**

(72) Inventor: **Wilson, Harve Clark**
**10525 Arend Road**
**Martinsville Indiana 46151(US)**

(74) Representative: **Tapping, Kenneth George et al,**
**Lilly Industries Limited Patent Department Erl Wood**
**Manor**
**Windlesham Surrey, GU20 6PH(GB)**

(54) **Inotropic agent.**

(57) 4-[3(R)-([2-hydroxy-3-(3-hydroxyphenoxy)propyl]-amino)-butyl]-benzamide and pharmaceutically acceptable salts thereof produce a positive inotropic effect in mammals.

EP 0 151 513 A1

**0151513**

## INOTROPIC AGENT

This invention provides an orally effective, long acting, positive inotropic agent useful for long term management of heart failure.

The cardiac glycosides and the sympathomimetic amines are the principal inotropic agents used in the management of congestive heart failure. Although the cardiac glycosides, especially digitalis, are among the most frequently prescribed drugs, they have numerous liabilities such as a low therapeutic index and erratic absorption, and are associated with life-threatening arrhythmias and deleterious drug-drug interactions. In addition, many patients either do not respond, or become refractory to these agents. The sympathomimetic amines, such as dopamine and epinephrine, have limited utility due to positive chronotropic effects, arrhythmogenic properties, and oral ineffectiveness.

Many propanolamine derivatives are known in the art to be β-adrenergic blocking agents useful in the treatment of a variety of cardiovascular conditions such as angina pectoris and hypertension. Certain propanolamines, such as some of those described in Canadian Patent No. 1,134,843 and U.S. Patents No. 4,027,027 and 4,244,969, are taught to possess β-stimulating or β-agonist activity thereby making the compounds useful as positive inotropic agents.

This invention provides the compound 4-[3(R)-([2-hydroxy-3-(3-hydroxyphenoxy)propyl]amino)butyl]-benzamide and pharmaceutically acceptable salts thereof. For purposes of this disclosure, the compound and its salts will hereinafter be referred to collectively as Compound I.

Compound I is an orally effective, long acting, positive inotropic agent which should be useful for the long term management of heart failure.

According to a further aspect of the present invention there is provided a pharmaceutical formulation which comprises as active ingredient Compound I associated with a pharmaceutically acceptable carrier.

Compound I contains two asymmetric centers. It is critical for this invention that the chiral center at the 3-position of the butylbenzamide moiety is in the R-configuration. The 2-hydroxy group of the propanolamine portion of the molecule may be in the R or preferably the S configuration or the compound may be employed as the mixture of the two isomers. Therefore, this invention includes both 4-[3(R)-([2(R)-hydroxy-3-(3-hydroxy-phenoxy)propyl]amino)butyl]benzamide and 4-[3(R)-([2(S)-hydroxy-3-(3-hydroxyphenoxy)propyl]amino)butyl]-benzamide as well as the mixture thereof.

The pharmaceutically acceptable acid addition salts of Compound I include salts derived from inorganic acids such as hydrochloric acid, nitric acid, phosphoric acid, sulfuric acid, hydrobromic acid, hydroiodic acid, phosphorous acid and the like, as well as salts derived from nontoxic organic acids such as aliphatic mono- and di-carboxylic acids, phenyl-substituted alkanoic acids, hydroxy-alkanoic and -alkanedioic acids, aromatic acids, aliphatic and aromatic sulfonic acids, etc. The preferred salts of Compound I are those derived from inorganic acids, especially hydrochloric acid.

Compound I can be prepared by any of a number of methods known in the art. These methods include, but are not limited by, the following reactions:

(a) The reaction of a 1-(3-blocked-hydroxyphenoxy)-2,3-epoxypropane with 4-(3-aminobutyl)benzamide;

(b) The reaction of a 1-(3-blocked-hydroxyphenoxy)-2-hydroxy-3-halopropane with 4-(3-aminobutyl)benzamide;

(c) The reaction of resorcinol with 4-(3-[(2,3-epoxypropyl)amino]butyl)-benzamide;

(d) The reaction of resorcinol with a 4-(3-[(3-halo-2-hydroxypropyl)amino]butyl)-benzamide;

(e) The reaction of a 3-(3-blocked-hydroxyphenoxy)-2-hydroxy-1-aminopropane with a 4-(3-halobutyl)benzamide; and

(f) The reaction of 3-(3-hydroxyphenoxy-2-hydroxy-1-aminopropane with 4-(4-carbamoylphenyl)-2-butanone under hydrogenation conditions.

In methods (a), (b), and (e) above, the term "blocked-hydroxy" refers to a hydroxy group which is blocked with a nonreactive functionality. These reactions provide "blocked" intermediates of Compound I which are then deblocked by a subsequent transformation. Although many such blocking groups are known to those skilled in the art, the preferred group for this process is the benzyl group which can be removed by hydrogenation. Hydrogenation is usually performed in the presence of a catalyst, such as Raney nickel or palladium on carbon, in a solvent such as methanol or ethanol.

In the reactions involving epoxide intermediates [methods (a) and (c)], the reaction between the two compounds is best performed with a slight molar excess of the epoxy intermediate. The reactions are usually heated at temperatures of 40-100°C., with or without the presence of a nonreactive solvent, such as dioxane, dimethylformamide, or an alcohol. The reaction is usually complete within 1-24 hours.

In reactions (b), (d), and (e), "halo" may be chloro, bromo, fluoro, or iodo, with chloro and bromo being preferred. The reactions are generally carried out in a nonreactive solvent such as dimethylformamide, dimethylsulfoxide, hexamethylphosphoramide, alcohols, and the like, preferably in the presence of an acid scavenger, such as sodium bicarbonate. The coupling reaction can be carried out at temperatures ranging from about 0°C. to the reflux temperature of the reaction mixture. A temperature of about 20-60°C. is preferred. The reactants are generally reacted in equimolar amounts although other ratios are operative.

Method (f) is a reductive amination procedure known in the art. The reaction of the two reactants results in the loss of water and the formation of an imine intermediate which is subsequently hydrogenated by standard methods to form the desired Compound I. This hydrogenation procedure introduces a new chiral center and generally yields a mixture of diastereomeric products unless a selective catalyst is used which can impart selective generation of a particular configuration.

As will be recognized by one skilled in the art, in methods (a)-(e), if one or both chiral centers of the reactant(s) are unresolved, the resulting intermediate and product will be a mixture of diastereomers which can then be resolved by methods known in the art, if desired. Alternatively, the reactant(s) may be first prepared or resolved as a single optically active isomer and then reacted to directly produce the corresponding optically active diastereomer of Compound I.

Variations on the above reaction schemes will be obvious to those skilled in the art. For instance, instead of using benzamide reagents, the corresponding benzonitrile intermediate may be employed producing the corresponding nitrile analog of Compound I. This nitrile compound is known in the art (See U.S. Patent No. 4,210,653, Example 6). The nitrile intermediate may then be hydrolyzed by standard methods to the desired benzamide Compound I. Alternatively, a benzoate ester of the corresponding benzamide intermediates may be employed producing the analogous benzoate of the desired product. This ester may then be subject to ammonolysis to prepare the desired benzamide compound.

Other reaction schemes are apparent from the literature. See generally U.S. Patents No. 4,195,090 and 4,018,824, and Canadian Patent No. 1,134,843. The intermediates required to prepare the compound of this invention are either known in the art or can be prepared by published methods.

Thus, one aspect of the invention is a process for preparing 4-[3(R)-([2-hydroxy-3-(3-hydroxyphenoxy)-propyl]amino)-butyl]benzamide, or a pharmaceutically acceptable salt thereof, which comprises reacting 4-(3(R)-aminobutyl)benzamide with 1-(3-blocked-hydroxyphenoxy)-2,3-epoxypropane, to produce a blocked intermediate, removing the blocking group, and, if desired, salifying the intermediate or the final product.

Compound I may be administered by various routes including the oral, rectal, transdermal, subcutaneous, sublingual, intravenous, intramuscular, or intranasal routes, being usually employed in the form of a pharmaceutical composition. It is a special feature that Compound I is an effective positive inotrope agent following oral administration. Such compositions are prepared in a manner well known in the pharmaceutical art and comprise at least one active compound. Accordingly, the invention includes pharmaceutical compositions comprising as active ingredient Compound I or a pharmaceutically acceptable salt thereof, associated with a pharmaceutically acceptable carrier.

In making the compositions of the present invention, Compound I will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the composition can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions and sterile packaged powders.

Some examples of suitable carriers are lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propylhydroxybenzoates, talc, magnesium stearate or mineral oil. The formulations can additionally include lubricating agents, wetting agents, emulsifying and suspending agents, preserving agents, sweetening agents or flavoring agents. The compositions of the invention may, as is well known in the art, be formulated so as to provide quick, sustained, or delayed release of the active ingredient after administration to the patient.

The compositions are preferably formulated in a unit dosage form, each dosage containing from about 1 to 50 mg., more usually 5 to 30 mg., of Compound I. The term "unit dosage form" refers to physically discrete units suitable as unitary dosages for human subjects and other mammals, each unit containing a predetermined quantity of Compound I calculated to produce the desired therapeutic effect, in association with the required pharmaceutical carrier.

Compound I is effective over a wide dosage range. For example, dosages per day will normally fall within the range of about 0.05 to 3 mg./kg. In the treatment of adult humans, the range of about 0.1 to 0.5 mg./kg., in single or divided doses, is preferred. However, it will be understood that the amount of the compound actually administered will be determined by a physician, in the light of the relevant circumstances including the chosen route of administration, and the age, weight, and response of the individual patient, and therefore the above dosage ranges are not intended to limit the scope of the invention in any way.

The following examples further illustrate the preparation of the compound and formulations of this invention. The examples are illustrative only and are not intended to limit the scope of the invention in any way.

## Example 1

4-[3(R)-([2-hydroxy-3-(3-hydroxyphenoxy)-propyl]amino)butyl]benzamide hydrochloride

A solution of 8.0 g. (.0418 mole) of 4-(3(R)-aminobutyl)- benzamide and 11.249 g. (.0439 mole) of 1-(3-benzyloxyphenoxy)-2,3- epoxypropane in 200 ml. of methanol was heated at reflux overnight. The reaction mixture was evaporated in vacuo and the residue was taken up in diethyl ether. After filtration, the ether solution was evaporated to dryness. The residue was dissolved in a minimum volume of ethanol and a large excess of water was added. The supernate was decanted off and the residual oil was dissolved in a small volume of acetonitrile. A minimum volume of hexane was added. The resulting solid was recovered by filtration and recrystallized from acetonitrile. The recrystallized material was treated with 25 ml. of diethyl ether which was saturated with anhydrous hydrogen chloride. After evaporation, the residue was dissolved in ethanol, evaporated, and then triturated with diethyl ether. The product was recrystallized from acetonitrile/methanol to provide 5.23 g. (.0117 mole) of the intermediate 4-[3(R)-([2-hydroxy-3-(3-benzyloxyphenoxy)propyl]amino)butyl]benzamide hydrochloride, m.p. 138.5-140°C.

Five and one-tenth grams of the above benzyloxy intermediate were treated with Raney nickel, methanol, and hydrogen gas under pressure until approximately 100% of the theoretical uptake was obtained. The catalyst was removed by filtration and the filtrate was evaporated in vacuo. Crystallization from acetonitrile/methanol provided 3.2 g. of the desired title product, m.p. 175.5-177.2°C.; $[\alpha]_D = +11.98°$, $[\alpha]_{365} = +43.31°$ [c = 10 mg./ml. methanol]; $^{13}C$ and proton NMR spectra were consistent with the structure of the title product.

Analysis: $C_{20}H_{26}N_2O_4$ HCl;
  Calc.: C, 60.83; H, 6.89; N, 7.09;
  Found: C, 60.80; H, 6.62; N, 7.16.

### Example 2

Hard gelatin capsules are prepared using the following ingredients:

|                      | Quantity (mg./capsules) |
| -------------------- | ----------------------- |
| Compound I           | 25                      |
| Starch dried         | 225                     |
| Magnesium stearate   | 10                      |

The above ingredients are mixed and filled into hard gelatin capsules in 260 mg. quantities.

## Example 3

A tablet formula is prepared using the ingredients below:

| | Quantity (mg./tablet) |
|---|---|
| Compound I | 25 |
| Cellulose, microcrystalline | 400 |
| Silicon dioxide, fumed | 10 |
| Stearic acid | 5 |

The components are blended and compressed to form tablets each weighing 440 mg.

## Example 4

An aerosol solution is prepared containing the following components:

| | Weight % |
|---|---|
| Compound I | 0.25 |
| Ethanol | 29.75 |
| Propellant 22 (Chlorodifluoromethane) | 70 |

Compound I is mixed with ethanol and the mixture added to a portion of the propellant 22, cooled to -30°C. and transferred to a filling device. The required amount is then fed to a stainless steel container and diluted further with the remainder of the propellant. The valve units are then fitted to the container.

X-6123 - 12 -

## Example 5

Tablets each containing 5 mg. of Compound I are made up as follows:

| | |
|---|---|
| Compound I | 5 mg. |
| Starch | 100 mg. |
| Microcrystalline cellulose | 35 mg. |
| Polyvinylpyrrolidone (as 10% solution in water) | 4 mg. |
| Sodium carboxymethyl starch | 4.5 mg. |
| Magnesium stearate | 0.5 mg. |
| Talc | 1 mg. |
| | -- |
| Total | 150 mg. |

The Compound I, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C. and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

X-6123                              - 13 -


## Example 6


Capsules each containing 20 mg. of compound I are made as follows:

| Compound I | 20 mg. |
|---|---|
| Starch | 89 mg. |
| Microcrystalline cellulose | 89 mg. |
| Magnesium stearate | 2 mg. |
| Total | 200 mg. |

The Compound I, cellulose, starch and magnesium stearate are blended, passed through a No. 45 mesh U.S. sieve, and filled into hard gelatin capsules in 200 mg. quantities.

## Example 7


Suppositories each containing 25 mg. of compound I are made as follows:

| Compound I | 25 mg. |
|---|---|
| Saturated fatty acid glycerides to | 2,000 mg. |

The Compound I is passed through a No. 60 mesh U.S. sieve and suspended in the saturated fatty acid glycerides previously melted using the minimum heat necessary. The mixture is then poured into a suppository mold of nominal 2 g. capacity and allowed to cool.

## Example 8

Suspensions each containing 5 mg. of compound I per 5 ml. dose are made as follows:

| | |
|---|---|
| Compound I | 5 mg. |
| Sodium carboxymethyl cellulose | 50 mg. |
| Syrup | 1.25 ml. |
| Benzoic acid solution | 0.10 ml. |
| Flavor | q.v. |
| Color | q.v. |
| Purified water to | 5 ml. |

The Compound I is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

Compound I is a long-acting positive inotropic agent which is effective when given orally. This inotrope activity was demonstrated in the following test systems.

PHENOBARBITAL ANESTHETIZED DOG STUDY

Mongrel dogs of either sex weighing 8-20 kg. were anesthetized with sodium thiopental (15 mg./kg., i.v.) and sodium phenobarbital (100 mg./kg., i.v.). A positive pressure pump provided ventilation through a cuffed endotracheal tube (18 strokes/minute, 20 ml./kg./stroke) and a heating pad kept the body temperature at 37-38°C. Both vagus nerves were sectioned

X-6123                              - 15 -

through an incision in the neck.  Arterial blood pressure was monitored using a Statham pressure transducer attached to a cannula filled with heparinized saline (16 units/ml.) inserted into the abdominal aorta via the right femoral artery.  A cannula connected to a three-way stopcock was inserted in the right femoral vein for drug administration.  The heart was exposed through the fifth right intercostal space and a precalibrated Walton-Brodie strain gauge arch was sutured on the right ventricle.  The strain gauge was adjusted to a tension of 50 gm. at systole and the Beckman Dynograph recorder was set so that 50 gm. tension produced a pen deflection of 10 mm.  Heart rate was derived electronically from the blood pressure pulse using a Beckman cardiotachometer coupler.

Isoproterenol hydrochloride was dissolved and injected in acidified (pH 6.0) water.  The doses of isoproterenol were 0.025, 0.05, 0.1 and 0.2 mcg./kg.  Compound I hydrochloride was dissolved and injected in sterile water.  Doses were given at 5 minute intervals.

An isoproterenol standard curve was obtained in each dog and responses to the compound were adjusted according to the isoproterenol sensitivity.  Results of this study are summarized in Table 1.

TABLE 1

Effect of Isoproterenol and Compound I in
the Phenobarbital Anesthetized Dog*

| Compound | Dose (mcg./kg., i.v.) | Arterial Pressure | Heart Rate | Cardiac Contractility |
|---|---|---|---|---|
| Isoproterenol | 0.025 | 90±4 | 108±2 | 147±12 |
| | 0.050 | 84±3 | 116±4 | 187±14 |
| | 0.100 | 78±4 | 126±5 | 235±19 |
| | 0.200 | 72±2 | 134±7 | 270±18 |
| Compound I hydrochloride | 0.125 | 99±1 | 101±1 | 116± 5 |
| | 0.375 | 100±1 | 103±1 | 141±12 |
| | 0.875 | 102±1 | 108±2 | 190±20 |
| | 1.875 | 102±1 | 115±3 | 239±28 |
| | 3.875 | 101±1 | 123±4 | 271±30 |
| | 7.875 | 100±3 | 129±3 | 281±32 |
| | 15.875 | 99±3 | 133±2 | 278±30 |
| | 31.875 | 104±6 | 131±3 | 259±48 |
| | 63.875 | 98±1 | 131±2 | 262±42 |

*results expressed as percent of control (control = 100%).
Values are the mean ± standard error of the mean of four experiments.

CONSCIOUS INSTRUMENTED DOG STUDY

Male mongrel dogs weighing 15.4 to 38.3 kg. were anesthetized with sodium pentobarbital (30 mg./kg., i.v.) and ventilated through a cuffed endotracheal tube (18 strokes/minute, 20 ml./kg./stroke) with a positive pressure pump. Body temperature was maintained at 37-38°C. with a heating pad. The heart was exposed through the left, fifth, intercostal space and a pre-calibrated Konigsberg implantable pressure transducer was positioned in the left ventricle through a stab wound in the apex of the heart. The transducer connector cable was led dorsally beneath the skin to a point 5 cm. anterior to the shoulder blades. The chest was closed and evacuated. Dogs received antibiotics and at least two weeks were allowed for recovery before testing.

Dogs were conditioned to the testing laboratory prior to the study. They were confined to a pen and restrained with a tether with sufficient freedom to either stand or lie during the study. The Konigsberg pressure transducer was connected to a Beckman R-611 recorder. Left ventricular pressure and calculated cardiac inotropic activity was monitored by measuring the first derivative of the left ventricular pressure tracing when pressure reached 60 mm Hg. Heart rate was recorded by means of a Beckman cardiotachometer coupler driven from the left ventricular pressure wave. After a 20-30 minute control period, dogs received a capsule containing either Compound I hydrochloride at 0.5 mg./kg. or placebo (lactose). Measurements were obtained between 0-12 hours after dosing. No significant differences were seen between the

X-6123                    - 18 -


placebo and Compound I in heart rate or arterial pressure. The comparative data for cardiac contractility is summarized in Table 2.

## TABLE 2

Effect of Compound I and Placebo on
Cardiac Contractility in the Conscious Instrumented Dog

| Time After Dosing (hours) | Effect on Contractility* | |
|---|---|---|
| | Compound I (HCl) (0.5 mg./kg., p.o.) | Placebo (lactose) |
| 0.25 | 98 $\pm$ 2 | 97 $\pm$ 2 |
| 0.50 | 111 $\pm$ 5[+] | 94 $\pm$ 3 |
| 0.75 | 109 $\pm$ 6 | 94 $\pm$ 3 |
| 1.0 | 115 $\pm$ 9[+] | 93 $\pm$ 1 |
| 1.5 | 118 $\pm$ 10[+] | 91 $\pm$ 2 |
| 2.0 | 120 $\pm$ 11[+] | 89 $\pm$ 2 |
| 2.5 | 123 $\pm$ 11[+] | 96 $\pm$ 1 |
| 3.0 | 118 $\pm$ 7[+] | 95 $\pm$ 2 |
| 4.0 | 120 $\pm$ 5[+] | 95 $\pm$ 3 |
| 5.0 | 115 $\pm$ 3[+] | 94 $\pm$ 4 |
| 6.0 | 122 $\pm$ 4[+] | 98 $\pm$ 3 |
| 7.0 | 117 $\pm$ 6[+] | 97 $\pm$ 2 |
| 8.0 | 113 $\pm$ 4[+] | 95 $\pm$ 3 |
| 9.0 | 116 $\pm$ 2[+] | 93 $\pm$ 1 |
| 10.0 | 112 $\pm$ 3[+] | 92 $\pm$ 1 |
| 11.0 | 111 $\pm$ 2[+] | 98 $\pm$ 1 |
| 12.0 | 108 $\pm$ 1[+] | 93 $\pm$ 2 |

*percent of 0 time control values (controls = 100%). Each value is the mean $\pm$ the standard error of the mean of four experiments.

[+]$p < 0.05$ compared to placebo using a paired two-tailed t-test.

## CLAIMS

1.    4-[3(R)-([2-hydroxy-3-(3-hydroxyphenoxy)-propyl]amino)butyl]benzamide or a pharmaceutically acceptable salt thereof.

2.    A compound of Claim 1 in hydrochloride salt form.

3.    4-[3(R)-([2-hydroxy-3-(3-hydroxyphenoxy)propyl]amino)-butyl]benzamide, or a pharmaceutically acceptable salt thereof for use as a positive inotropic agent.

4.    A pharmaceutical formulation which comprises a compound of Claim 1 and a pharmaceutically acceptable carrier or diluent.

5.    A composition of Claim 4 wherein the compound is 4-[3(R)-([2-hydroxy-3-(3-hydroxyphenoxy)-propyl]amino)butyl]benzamide hydrochloride.

6.    A formulation of Claim 4 .or 5 which is formulated for oral administration.

**European Patent Office**

**EUROPEAN SEARCH REPORT**

Application number

EP 85 30 0197

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-1 957 706 (PFIZER) * Claims; pages 1,8,26 * | 1,4-6 | C 07 C 103/28 A 61 K 31/165 |
| Y | EP-A-0 007 204 (ELI LILLY) * Claims * | 1,4-6 | |
| Y | EP-A-0 099 707 (BEECHAM) * Claims * | 1,4-6 | |
| D,Y | EP-A-0 006 614 (MERCK) * Claims; pages 1,14 * | 1,4-6 | |
| | | | TECHNICAL FIELDS SEARCHED (Int. Cl.4) |
| | | | C 07 C 103/00 A 61 K 31/00 |

The present search report has been drawn up for all claims

| Place of search | Date of completion of the search | Examiner |
|---|---|---|
| THE HAGUE | 19-04-1985 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82